(19) Europäisches Patentamt
European Patent Office
Office européen des brevets

(11) EP 3 449 901 A1

(12) **EUROPEAN PATENT APPLICATION**

(43) Date of publication:
**06.03.2019 Bulletin 2019/10**

(21) Application number: **17382594.4**

(22) Date of filing: **04.09.2017**

(51) Int Cl.:
***A61K 8/64*** *(2006.01)* ***A61Q 19/08*** *(2006.01)*
***A61K 8/97*** *(2017.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Dermofarm, S.A.**
**08191 Rubi (ES)**

(72) Inventor: **GODIA OCHOA, Juan**
**08191 RUBI (ES)**

(74) Representative: **Ponti & Partners, S.L.P**
**C. de Consell de Cent 322**
**08007 Barcelona (ES)**

(54) **COSMETIC OR DERMOCOSMETIC COMPOSITION, PROCESS FOR ITS PREPARATION AND USE THEREOF**

(57) The present invention relates to a cosmetic or dermocosmetic composition comprising the following ingredients: extract of apple (*Malus domestica)* stem cells; extract of grape (*Solar vitis*) stem cells; at least one pentapeptide; a complex consisting of: epidermal growth factor, insulin-like growth factor, fibroblast growth factor (acid and basic), vascular endothelial growth factor, vitamin B9 and glutamine. The present invention further relates to a product comprising said composition, to a process for preparing said composition or said product and to the use of said composition or said product for treating the aging of the skin.



Printed by Jouve, 75001 PARIS (FR)

## Description

Field of the Invention

The present invention relates to the cosmetic filed. In particular, the present invention relates to a cosmetic or dermocosmetic composition useful for treating the aging of the skin.

Background of the invention

Aging corresponds to the set of physiological and psychological processes that alter the structure and functions of the body after a certain age. There are two types of aging, namely, intrinsic aging on the one hand, and extrinsic aging on the other hand. Intrinsic aging is due to genetic factors, biochemical changes that occur during conditions of fatigue, stress, hormonal changes such as during pregnancy, etc. Extrinsic aging, on the other hand, is caused by to environmental factors to which the body is subjected throughout life, such as pollution, sun, diseases, etc. This is a slow and gradual process that affects every cell in the body by various means and manifests itself in different ways. For example, in the case of the skin, its appearance is modified by various types of internal or external aggression, and the emergence of wrinkles and fine lines, hyper- or hypo-pigmentation spots, dryness or even dehydration of the skin, thinning of the skin, elastosis, blemishes, age spots, etc... can be observed.

In mammals and especially humans, the skin and eyes are organs that are subjected to continuous renewal. For example, the skin surface desquamation phenomenon must be compensated for by epidermis renewal, which is ensured by the keratinocytes in the basal layer, which actively divide and differentiate into cells of the stratum corneum.

Epidermal stem cells (ESC) are cells that have an unlimited capacity for renewal, being able to constitute a complete epidermis with all its layers. During aging there is a weakening and loss of stem cells that affects all layers of the skin. This fact is favored by UV radiation, free radicals,.... These cells act as a cellular reserve helping in the processes of renewal and skin repair: when a stem cell is lost millions of cells are not renewed, which causes the skin to age, lose elasticity, appear wrinkles and become flaccid and dull.

Anti-aging compositions are well known in the art. Nevertheless, there is an on-going need for improved anti-aging compositions, in particular for compositions with better properties such as elasticity, firmness, hydration and with a wrinkle reduction.

In this regard, the present inventors have developed a cosmetic or dermocosmetic composition which provides a global anti-aging solution for both intrinsic and extrinsic damages (such as photo-aging produced by UV radiation), increasing the longevity of epidermal stem cells as well as maintaining their regeneration power, protecting them free radicals, preserving their micro-environment and the factors that regulate their fate and increasing the synthesis of energy to improve the adaptive response to changes.

Such a solution is based on a complex of actives, a mixture of growth factors, stem cell extracts and pentapeptides whose actions are complemented and surprisingly enhanced, and are able to protect and optimize the environment surrounding the skin stem cells by protecting them and stimulating the formation of new cells that will be able to counteract and repair the effects of the passage of time and give the skin a youthful and healthy appearance.

The epidermal growth factor (EGF), like the other known growth factors, is of great importance in cell physiology, both in its maturation and metabolic and repair functions. Its discovery opened a new era in cell biology. In the skin there are EGF receptors on the dermis, epidermis and hair, and the response is not only mitogenic (proliferation of fibroblasts and keratinocytes) but also a non-mitogenic response favoring the synthesis of collagen, intercellular matrix, hyaluronic acid formation, is also present, acting also on the superoxide dismutase, having thereby an antioxidant action. The EGF can then be used in the treatment of cutaneous aging because:

- it regulates cell function in changes produced by keratinocytes, which contributes to epidermal repair and renewal;
- it increases the consistency of the skin and its hydration by increasing the hyaluronic acid in the intercellular matrix by activating fibroblast function;
- ultraviolet rays cause injury (qualitative and quantitative) to the cellular receptors of EFG. Treatment with EGF contributes to cell repair of sun-induced damage;
- it indirectly has an antioxidant action by increasing the synthesis of superoxide dismutase (SOD)
- it improves the transport system of macromolecules and ions to the inner of the cell by activating the calcium-dependent membrane systems.

As developed below, the present inventors have surprisingly found that combining growth factors with particular ingredients, the anti-aging effect is clearly enhanced.

Brief description of drawings

Figure 1 shows melting curves showing a single peak for all the oligos used in the qPCR assay according to example 4.

Figures 2(A,B,C) show *the parameters evaluated to check technical quality of the qRT-PCR reaction. Amplification plot vs cycle represent the magnitude of normalized fluorescence signal generated by the reporter at each cycle during PCR amplification; in plot amplification Ct vs Well, Ct indicates the PCR cycle number at which the fluorescence meets the threshold in the amplification plot; good Ct values should stay in the range >8-35<; the plot Fluorescence vs Cycle indicates the fluorescence signal from the reporter dye normalized to the fluorescence signal from the passive reference. All these parameters allow identifying and examining irregularity in the amplification.*

Figure 3. *Bar graphs showing COL1 expression results after treating Normal Human Dermal Fibroblasts (NHDF) with Origin Pro EGF-5 at 0.01 %, 0.05 % and 0.1 % during 24 hours. ** Represents statistical significance with p value < 0.01. **** Represents statistical significance with p value < 0.0001.*

Figure 4. Bar graphs showing ELN expression results after treating Normal Human Dermal Fibroblasts (NHDF) with Origin Pro EGF-5 at 0.05 % and 0.1 % during 24 hours. * Represents statistical significance with p value < 0.05.

Figure 5 shows the % reduction of ROS at different sample concentrations and different times of UV exposure.

Summary of the invention

A first object of the present invention is to provide a cosmetic or dermocosmetic composition overcoming the above mentioned drawbacks.
A second object of the present invention is to provide a product comprising the composition according to the first object of the invention.
A third object of the present invention is to provide a process for preparing the composition according to the first object of the invention.
A fourth object of the present invention is to provide a process for preparing the product according to the second object of the invention.
A fifth object of the invention is to provide a use for the composition according to the first object of the invention or the product according to the second object of the invention for treating the aging of the skin.

Detailed description of the invention

In a first aspect, the present invention relates to a cosmetic or dermocosmetic composition comprising the following ingredients:

- extract of apple *(Malus domestica)* stem cells;
- extract of grape *(Solar vitis)* stem cells;
- at least one pentapeptide;
- a complex consisting of: epidermal growth factor, insulin-like growth factor, fibroblast growth factor (acid and basic), vascular endothelial growth factor, vitamin B9 and glutamine.

The term "cosmetic" generally refers to (i) a substance intended to be rubbed, poured, sprinkled or sprayed on, introduced, or otherwise applied to the human body or other animal body or any part thereof for cleansing, beautifying, promoting attractiveness, or altering the appearance, and (ii) a substance intended for use as a component of any such substances, except that such term shall not include soap. Non-limiting examples of products included in this definition are skin moisturizers, eye and facial preparations, skin lighteners, masques, lotions, toners, and any material intended for use as a component of a skin cosmetic product. Generally, such products may be applied to large surface areas of skin, to damaged skin, and to delicate areas of the skin, and are left on the skin for long periods of time. In the context of the present invention a cosmetic product is to be employed to counteract the visible effects of aging skin.
The term "dermocosmetic" means in the context of the present invention a cosmetic composition intended for application on the skin.
The term "aging" in the context of the present invention refers to the changes experienced by the skin with age (chronoaging) or through exposure to the sun (photoaging) or to environmental agents such as tobacco smoke, extreme climatic conditions of cold or wind, chemical contaminants or pollutants, and includes all the external visible and/or perceptible changes through touch, such as and not restricted to, the development of discontinuities on the skin such as wrinkles, fine lines, expression lines, stretch marks, striae, furrows, irregularities or roughness, increase in the size of pores, loss of moisture, loss of elasticity, loss of firmness, loss of smoothness, loss of the capacity to recover from deformation, loss of resilience, sagging of the skin such as sagging cheeks, the appearance of bags under the eyes or the appearance of a double chin, among others, changes to the color of the skin such as marks, reddening, bags or the appearance of hyperpigmented areas such as age spots or freckles among others, anomalous differentiation, hyperkeratinization, elastosis, keratosis, hair loss, orange peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and it presents the same physical characteristics as aging, such as and not restricted to, flaccidity, sagging, changes to the color or irregularities in the pigmentation, abnormal and/or excessive keratinization. The sum of different environmental factors such as exposure to tobacco smoke, exposure to pollution, and climatic conditions such as cold and/or wind also contributes to the aging of the skin.

In a preferred embodiment, said at least one pentapeptide is pentapeptide-31 or pentapeptide-28 or a combination thereof.

In another preferred embodiment, the above mentioned ingredients of the cosmetic or dermocosmetic composition are present in the following weight ratio (with respect to the total composition):

- extract of apple (*Malus domestica)* stem cells between 0.2 and 5 wt%;
- extract of grape (*Solar vitis*) stem cells between 0.2 and 5 wt%;
- at least one pentapeptide between 0.2 and 5 wt% for each pentapeptide;
- the complex between 0.2 and 5 wt%.

In another preferred embodiment, said cosmetic or dermocosmetic composition further comprises at least one of the following ingredients:

- apricot extract, which is rich in phenolic compounds and Vitamin C, confers antioxidant and clarifying properties, and also rich in carbohydrates and alpha-hydroxy acids which confers exfoliating properties, contributing to the renewal of the layer and unifying and clarifying the skin tone;
- panthenol (vitamin B5), which activates the cellular regeneration and has antiinflammatory action, lowers itching and reduces redness and signs of inflammation of irritated skin;
- lotus flower extract, which is rich in essential fatty acids Omega 6 and Omega 9, palmitic acid and stearic acid and cholesterol precursors (squalene), members of the intercellular cement that regulate the Insensible Water Loss (IWL) and take part in the intercorneocitary cohesion and in the process of desquamation and contributes to a fast restoration of the barrier function, avoiding thereby the dehydration (in particular, the lotus flower extract can be pink lotus flower extract which is rich in starch, vitamin C, asparagine and nelumbina, and has antioxidant and stimulant action of the cutaneous microcirculation);
- hyaluronic acid, which is a natural component of the dermis and contributes, along with the collagen, elastin and basic substance, to the hydration, elasticity and firmness of the skin. Due to its great affinity for water, it forms a viscoelastic gel which lowers the Insensible Water Loss (IWL) and favors its uptake. It also increases hydration of the skin and smoothes the skin relief and eliminates signs of fatigue and improves skin tone and elasticity.
- viola tricolor extract, which is rich in oligosaccharides, contributes to the circulation of water between dermis and epidermis by stimulating the synthesis of Aquaporin P3, a skin specific protein, and increases the water retention capacity in the epidermis by stimulating the synthesis of hyaluronic acid;
- polyoses from oats extract which have the property of forming a soft, resistant and elastic film with an immediate and perceptible tensor effect and a long-term anti-wrinkle action.

The composition according to the invention can also comprise any cosmetically acceptable medium or ingredient.

In a second aspect, the present invention relates to a product comprising the cosmetic or dermocosmetic composition according to the first aspect of the invention, including all the possible embodiments.

In a preferred embodiment, said cosmetic or dermocosmetic composition according to the first aspect of the invention is present in a concentration between 0.01% and 5% by weight, preferably between 0.01% and 1% by weight, more preferably between 0,01% and 0.1% by weight, with respect to the total weight of the product.

In another preferred embodiment, said product is in the form of an emulsion with oil as the external phase (W/O).

In another preferred embodiment, said product is in the form of an oil-in-water dispersion without emulsifier.

In another preferred embodiment, said product is in the form of an emulsion with water as the external phase (O/W).

In another preferred embodiment, said product is in the form of a solution.

In another preferred embodiment, said product is in the form of a gel.

In a third aspect, the present invention relates to a process for preparing the cosmetic or dermocosmetic composition, according to the first aspect of the invention, including all the possible embodiments, comprising the step of mixing all the ingredients with simple and/or fast agitation.

In a fourth aspect, the present invention relates to a process for preparing the product according to the second aspect of the invention, including all the possible embodiments.

In a preferred embodiment, the process for preparing the product in the form of an emulsion with oil as the external phase (W/O) comprises the steps of:

a) heating the oil phase at 75-80°C,
b) heating the aqueous phase at 75-80°C,
c) pouring the aqueous phase over the oil phase with agitation and shear;
d) cooling with slow agitation.

In another preferred embodiment, the process for preparing the product in the form of an oil-in-water dispersion without emulsifier or in the form of an emulsion with water as the external phase (O/W), comprises the steps of:

a) heating the aqueous phase at 75-80°C,
b) heating the oil phase at 75-80°C,
c) pouring the oil phase over the aqueous phase with agitation and shear;
d) cooling with slow agitation.

In a fifth aspect, the present invention relates to the use of the composition, according to the first aspect of the invention, including all the possible embodiments, or the product, according to the second aspect of the invention, including all the possible embodiments, for treating the aging of the skin.

The following Examples are offered for illustrative purposes only and are not intended to limit the scope of the present invention in any way.

**EXAMPLES**

Example 1. Anti-aging cream

The composition, with the components and ratios shown in table 1, was obtained as an emulsion with oil as the external phase (W/O). In a reactor the oil phase ingredients were weighted and heated to 75-80°C. In a different reactor or container the aqueous phase ingredients were weighted and heated also to 75-80°C. The mixture or emulsion is obtained by pouring the aqueous phase over the oil phase by applying agitation and shear. The thus obtained emulsion is then cooled under slow agitation and has the characteristics of a fresh and fluid texture and it is easily spreadable and absorbable.

Table 1.

| **Ingredients** | **CREAM** | **wt %** |
|---|---|---|
| Glycerin | | 8.000 |
| Panthenol | | 0.500 |
| Hyaluronic acid | | 0.030 |
| Vitamin E | | 1.000 |
| Oxynex K® liquid | | 0.100 |
| Osilift® | | 2.000 |
| Survixyl IS® | | 0.500 |
| Chondricare IS® | | 0.500 |
| Bio-placenta | | 1.000 |
| PhytocellTech™ Vitis | | 0.400 |
| PhytocellTech™ Malus Domestica | | 1.000 |
| Apricot extract HGL | | 3.000 |

(continued)

| Ingredients | CREAM | wt % |
|---|---|---|
| Aquaphyline EL® | | 0.020 |
| Pink lotus flower extract | | 2.000 |
| TOTAL OF ACTIVES | | 20.050 |

Bio-placenta corresponds to the complex obtained by microbial biofermentation containing five cellular growth factors (epidermal growth factor, insulin-like growth factor, acid fibroblast growth factor, basic fibroblast growth factor, vascular endothelial growth factor), vitamin B9 (also known as folic acid) and glutamine.

Example 2. Anti-aging serum

The composition, with the components and ratios shown in table 2, was obtained as a bigel, i.e a dispersion of oil-in-water without emulsifier. In a reactor the aqueous phase ingredients were weighted and heated to 75-80°C. In a different reactor or container the oil phase ingredients were weighted and heated also to 75-80°C. The dispersion is obtained by pouring the oil phase over the aqueous phase by applying agitation and shear. The thus obtained dispersion is then cooled under slow agitation and has the characteristics of a fresh and fluid texture and it is easily spreadable and absorbable.

Table 2.

| Ingredients | SERUM | wt% |
|---|---|---|
| Glycerin | | 8.000 |
| Panthenol | | 0.700 |
| Hyaluronic acid | | 0.050 |
| Vitamin E | | 1.000 |
| Oxynex K®) liquid | | 0.100 |
| Osilift® | | 4.000 |
| Survixyl IS® | | 0.750 |
| Chondricare IS® | | 1.000 |
| Bio-placenta | | 1.000 |
| PhytocellTech™ Vitis | | 0.400 |
| PhytocellTech™ Malus domestica | | 1.000 |
| Apricot extract HGL | | 7.000 |
| Ginko Bilboa Incloro MS extract HG | | 7.000 |
| Aquaphyline EL® | | 0.050 |
| Pink lotus flower extract | | 1.000 |
| TOTAL OF ACTIVES | | 33.050 |

Example 3. Anti-aging eye contour cream

The composition, with the components and ratios shown in table 3, was obtained as an emulsion with water as the external phase (O/W). In a reactor the aqueous phase ingredients were weighted and heated to 75-80°C. In a different reactor or container the oil phase ingredients were weighted and heated also to 75-80°C. The mixture or emulsion is obtained by pouring the oil phase over the aqueous phase by applying agitation and shear. The thus obtained emulsion is then cooled under slow agitation and has the characteristics of a fresh and fluid texture and it is easily spreadable and absorbable.

Table 3.

| Ingredients | EYE CONTOUR | wt% |
|---|---|---|
| Glycerin | | 6.000 |
| Panthenol | | 0.500 |

(continued)

| Ingredients | EYE CONTOUR | wt% |
|---|---|---|
| Hyaluronic acid | | 0.030 |
| Vitamin E | | 1.000 |
| Oxynex K® liquid | | 0.200 |
| Osilift® | | 4.000 |
| Survixyl IS® | | 0.500 |
| Chondricare IS® | | 1.000 |
| Bio-placenta | | 1.000 |
| PhytocellTech™ Vitis | | 0.200 |
| PhytocellTech™ Malus domestica | | 0.500 |
| HALOXYL™ | | 1.500 |
| EYELISS™ | | 0.800 |
| Apricot extract HGL | | 7.000 |
| Ginko Bilboa Incloro MS extract HG | | 6.300 |
| Aquaphyline EL® | | 0.020 |
| Pink lotus flower extract | | 3.000 |
| **TOTAL OF ACTIVES** | | 33.050 |

Example 4. Analysis of the effects of the composition of example 2

Much of the skin aging occurs in the dermis, which is mostly composed of a dense, collagen-rich extracellular matrix (ECM) that provides structure and support for the skin cells and confers tensile strength and firmness to the skin. Within the dermal ECM, aging is associated with a thickening of collagen fibrils and disorganization of total collagen content, mainly due to decreased collagen I synthesis and increased fibril fragmentation [Uitto, J. The role of elastin and collagen in cutaneous aging: intrinsic aging versus photoexposure. J Drugs Dermatol. 2008; 7 (2 supple.): S12-S16]. In addition, skin aging is associated with increased levels of matrix metalloproteinases, which can break down collagen and elastin fibers, combined with impaired transforming growth factor (TGF)-β signalling, which may reduce collagen deposition [Sardy, M. Role of matrix metalloproteinases in skin ageing. Connect Tissue Res. 2009; 50(2): 132-138].

Type I collagen is the most abundant collagen of the human body, which forms large, eosinophilic fibers known as collagen fibers. It is present in scar tissue, as well as tendons, ligaments, the endomysium of myofibrils, the organic part of bone, the dermis, the dentin and organ capsules. Elastin is a highly elastic protein in connective tissue and allows many tissues in the body to resume their shape after stretching or contracting. Elastin helps skin to return to its original position when it is poked or pinched. Elastin is also an important load-bearing tissue in the bodies or vertebrates and is used in places where mechanical energy is required to be stored.

For these reasons, in this assay, we assess the capacity of the composition of the present invention to increase the expression of Collagen 1 (COL1) and Elastin (ELN), after treatment on Normal Human Dermal Fibroblasts (NHDF) during 24 hours.

*4.1 Materials*

*4.1.1 Analytical equipment*

Microscope, incubator, refrigerated centrifuge, statistical analysis software, micropipettes, pipettes, propipette, rack, quantifier Nano-Drop spectrophotometer, laminar flow hood, ABI-ViiA 7 (Applied Biosystem) Quantitative real-time PCR, vortex, Bürker chamber, heating block, thermocycler and consumables.

*4.1.2 Reagents*

Braun Distilled water (Melsungen, Germany), primary fibroblast specific growth medium and supplement , Penicillin/Streptomicine (Life Technologies), Phosphate buffered saline (Sigma), Trypan Blue Solution (Bio-Rad), DNAse-I (Qiagen), RNeasy extraction kit (Qiagen), PrimeScript RT reagent kit (Perfect Real Time-Takara Clontech), oligonucleotides for RT-PCR amplification of *COL1, ELN,* and *ACT,* SYBR ®, qRT-PCR, liquid nitrogen, Lipopolysaccharide (Sigma).

*4.2 Procedure*

In order to perform cell count of live cells, cell viability was checked by staining with Trypan-Blue Solution (the blue compound can only penetrate in dead cells). Live cells were counted in a Burker chamber under the microscope.

For the main gene expression assay, NHDF cells were cultured at a 100.000 cells/well density in a 12 well plate, in growth media. 48 hours later, the culture media was replaced with new culture medium supplied with Origin Pro EGF-5, at 0.01 %, 0.05 % and 0.1 % concentrations (selected from the MTT assay), and for the untreated control samples were maintained in culture medium. After 24 hours of incubation, the medium was removed and cells were washed with PBS. Cells were then collected in lysis buffer to proceed with RNA extraction. Total RNA was extracted using miRNeasy kit (Qiagen) and treated with DNAse-I to remove any contamination from genomic DNA. RNA quality and quantity were checked in a Nano-Drop spectrophotometer, and 500 ng of total RNA was used to synthesize cDNA. The suitability of each primer pair used in this study for RT-qPCR, *ACT, COL1* and *ELN,* was previously evaluated to determine melting curves, efficiency of amplification and specificity of the primers. Finally, quantitative PCR was performed in a real time PCR machine (Applied BioSystem Viia7).

To perform raw data analysis, we used the Pfaffl method [Pfaffl, M. A new mathematical model for relative quantification in real-time RT-PCR. Nucleic Acids Research, 2001, Vo. 29 No. 9] to calculate the gene relative expression ratio to *ACT* (internal control-housekeeping gene). Mathematical model of relative expression ratio in real-time PCR is shown below. Statistical analysis to determine significant changes was performed using two-tails Student-*t test.* For all data a level of 5% or less ($p < 0.05$) was taken as statistically significant.

$$\text{ratio} = \frac{(E_{\text{target}})^{\Delta \text{CP}_{\text{target}}(control - sample)}}{(E_{\text{ref}})^{\Delta \text{CP}_{\text{ref}}(control - sample)}}$$

wherein the ratio of a target gene is expressed in a sample versus a control in comparison to a reference gene. Etarget is the real-time PCR efficiency of target gene transcript; Eref is the real-time PCR efficiency of a reference gene transcript; ΔCPtarget is the CP deviation control - sample of the target gene transcript; ΔCPref = CP deviation of control - sample of reference gene transcript.

*4.3 Results*

*4.3.1 Primer pair validation*

To evaluate the correct amplification and specificity of primer pairs for the genes of interest, melting curves for each primer pair were performed. Results showed an efficient amplification of the genes, with a single peak in the melting point, indicating the high specificity of the primers and their suitability for real-time application, as they do not form primer-dimer structures. The melting curves for each primer pair used in the assay are shown below. As shown, all the oligos amplify one amplicon and the melting temperatures are in the same range.

Figure 1 shows melting curves showing a single peak for all the oligos used in this qPCR assay. The correspondence in the range of melting temperature between targets and reference-gene is a parameter that indicates that data obtained are comparable.

Figure 2(A,B,C) shows a plot of the technical parameters taken into account for the analysis of the data. All together the parameters indicate that the efficiency of the PCR reaction was optimal.

*4.3.2 COL1 expression quantification by qPCR*

mRNA expression levels were determined for the product tested in Normal Human Dermal Fibroblasts (NHDF) after treatment during 24 hours. *COL1, ELN* and *Actin* (internal control) were amplified using four technical replicates of cDNAs.

Results showed that composition of example 2 at 0.01 % and 0.05 % significantly increased COL1 expression on Normal Human Dermal Fibroblasts by 34.2 ± 5.1 % and 17.8 ± 4.8 %, respectively. On the other hand, the treatment at 0.1 % increased COL1 expression by 7.8 ± 4.8 %, even though results were not statistically significant, compared to the untreated control; as shown in Figure 3 and Table 4.

Table 4: Statistical analysis of results shown in figure 3

| Table Analyzed | COL1 | COL1 | COL1 |
|---|---|---|---|
| | | | |
| Column B | 0.01% | 0.05% | 0.1% |
| vs. | vs. | vs. | vs. |
| Column A | Control | Control | Control |
| | | | |
| Unpaired t test | | | |
| P value | < 0.0001 | 0.0011 | 0.1135 |
| P value summary 0 | **** | ** | ns |
| Significantly different? (P < 0.05) | **Yes** | **Yes** | No |
| One- or two-tailed P value? | Two-tailed | Two-tailed | Two-tailed |
| t, df | t = 6.669 df = 28 | t = 3.683 df = 25 | t = 1.634 df = 28 |
| | | | |
| How big is the difference? | | | |
| Mean ± SEM of column A | 1.011 ± 0.03230 N = 13 | 1.011 ± 0.03230 N = 13 | 1.011 ± 0.03230 N = 13 |
| Mean ± SEM of column B | 1.353 ± 0.03737 N = 17 | 1.188 ± 0.03544 N = 14 | 1.089 ± 0.03381 N = 17 |
| Difference between means | **0.3421 ± 0.05129** | **0.1775 ± 0.04819** | 0.07829 ± 0.04792 |
| 95% confidence interval | 0.2370 to 0.4471 | 0.07825 to 0.2767 | -0.01987 to 0.1765 |
| R square | 0.6136 | 0.3518 | 0.08703 |

*4.3.3 ELN expression quantification by qPCR*

Results showed that composition of example 2 at 0.1 % significantly increased ELN expression on Normal Human Dermal Fibroblasts by 47.6 ± 15.8 %. On the other hand, the treatment at 0.05 % increased ELN expression by 13.6 ± 35.6 %, even though results were not statistically significant, compared to the untreated control; as shown in Figure 4 and Table 5. Samples from 0.01 % concentration were not properly amplified and results are not shown.

Table 5. Statistical analysis of results shown in figure 4

| Table Analyzed | ELN | ELN |
|---|---|---|
| | | |
| Column C | 0.05% | 0.1% |
| vs. | vs. | vs. |
| Column A | Control | Control |
| | | |
| Unpaired t test | | |
| P value | 0.7127 | 0.0166 |
| P value summary 0 | ns | * |
| Significantly different? (P < 0.05) | No | **Yes** |
| One- or two-tailed P value? | Two-tailed | Two-tailed |
| t, df | t=0.3817df=8 | t = 3.018 df = 8 |
| | | |

(continued)

| Table Analyzed | ELN | ELN |
|---|---|---|
| How big is the difference? | | |
| Mean ± SEM of column A | 1.013 ± 0.09381 N = 4 | 1.013 ± 0.09381 N = 4 |
| Mean ± SEM of column B | 1.149 ± 0.2789 N=6 | 1.488 ± 0.1112 N=6 |
| Difference between means | 0.1360 ± 0.3564 | **0.4755 ± 0.1575** |
| 95% confidence interval | -0.6859 to 0.9580 | 0.1122 to 0.8388 |
| R square | 0.01788 | 0.5325 |

*4.4 Discussion and Conclusions*

Skin aging is a natural process caused by both intrinsic changes and extrinsic damage. Much of the change occurs in the dermis, which is mostly composed of a dense, collagen-rich extracellular matrix (ECM) that provides structure and support for the skin cells and confers tensile strength and firmness to the skin. Within the dermal ECM, aging is associated with a thickening of collagen fibrils and disorganization of total collagen content, mainly due to decreased collagen I synthesis and increased fibril fragmentation. In addition, skin aging is associated with increased levels of matrix metalloproteinases, which can break down collagen and elastin fibers, combined with impaired transforming growth factor (TGF)-β signalling, which may reduce collagen deposition.

In this assay, we assess the capacity of composition of example 2 to increase the expression of Collagen 1 (COL1) and Elastin (ELN), after treatment on Normal Human Dermal Fibroblasts (NHDF) during 24 hours.

Results showed that composition of example 2 at 0.01 % and 0.05 % significantly increased COL1 expression on Normal Human Dermal Fibroblasts by 34.2 ± 5.1 % and 17.8 ± 4.8 %, respectively. In parallel, results showed that composition of example 2 at 0.1 % significantly increased ELN expression by 47.6 ± 15.8 %, compared to the untreated control.

The treatment with composition of example 2 during 24 hours on Normal Human Dermal Fibroblasts (NHDF) *in vitro,* significantly increased gene expression of Collagen 1 and Elastin, compared to the untreated control.

Example 5 In vitro evaluation of antioxidant activity of a cosmetic product (antiaging test)

Reactive oxygen species (ROS) are chemically reactive chemical species containing oxygen. Mitochondria are major generators of ROS in cells and tissues. ROS include, predominantly, the superoxide anion ($\bullet O_2$), hydrogen peroxide ($H_2O_2$), and the hydroxyl radical ($\bullet OH^-$). In a biological context, ROS are formed as a natural by-product of the normal metabolism of oxygen and have important roles in cell signaling and homeostasis. However, due to environmental stress (e.g., UV or heat exposure), ROS levels can increase dramatically.

ROS production is strictly correlated with the process of skin aging: the accumulation of ROS produced by normal metabolism - and the consequent cellular damage - is responsible for the intrinsic aging. On the other hand, extrinsic aging is caused by harmful free radicals created by environmental factors, including sun exposure. These radicals induce damage to the skin by causing an inflammatory reaction. Moreover, ROS also exacerbate enzyme activity from tyrosinase (main responsible for the melanin synthesis) and elastase (a proteolytic enzyme involved in the degradation of elastin). The result is the appearance of dark spot (mainly on the face and hands that are mostly exposed to sunlight) and the loss of skin elasticity. In particular sun exposure (ultraviolet) is known as a major cause of aging: among all the environmental factors, UV radiation contributes up to 80%. It is the most important factor in skin aging, especially in premature aging.

Owing to these reasons, if a tested product is able to reduce ROS production in vitro, it is also a good antiage candidate, potentially able to counteract and delay the process of skin aging. The aim of the following test is therefore to assess whether the tested product, at different concentrations, has antioxidant activity in vitro. For this reason we tested its efficacy in preventing the formation of reactive oxygen species (ROS), main responsible of skin aging. It is believed that this ability makes the product a potential antiaging candidate in vivo, able to counteract and delay the process of skin aging.

*5.1 Materials*

5.1.1 Cell line and culture conditions

The test was performed on human keratinocytes (Huker) cultured in DMEM (Dulbecco's modified Eagle medium) supplemented with 10% fetal bovine serum (FBS) and 1% antibiotics (penicillin and streptomycin) and incubated at standard culture conditions (37°C, 5% $CO_2$). Good cell culture practices were used.

*5.1.2 Irradiation conditions*

In order to simulate an environmental stress condition able to induce the production of ROS, cells were irradiated with UVA rays. The lamp used in the experiments is a solar light simulator. A constant emission in the UVA range (315-400 nm) with an irradiance of 1.7 mW/cm2 is assured. The UVB emission is screened in order to avoid direct cell mortality. We evaluated the effect on ROS production after 4, 8, 12, 16 and 20 minutes of irradiation.

*5.1.3 Tested sample*

The tested sample was the composition of the present invention in the form of a serum (example 2). The tested sample was dissolved in PEG and then diluted in culture medium to the desired final concentrations between 0.0156 and 1.0 mg/ml (1:2 dilutions). The negative control is represented by untreated cells, while as positive control (C+) we used a well known antioxidant. The 2',7'-dichlorodihydrofluorescein diacetate (DCFDA) was dissolved in dimethyl sulfoxide (DMSO) at the concentration of 50 mM and then diluted in the appropriate buffer at the final concentration of 250 μM.

*5.2 ROS assay*

An appropriate number of cells was seeded in 96-well plates. Once reached a semi-confluent monolayer, the cells were treated with different concentrations of the tested sample and the positive control, and incubated for 24 hours at standard culture conditions. The following day the cells were washed with phosphate buffered saline (PBS) and then incubated with the DCFDA solution for 20 minutes under standard culture conditions. The DCFDA was then removed, the cells were washed twice in PBS and then irradiated with UVA for 4, 8, 12, 16 and 20 minutes. At the end of each period of irradiation the fluorescence was read with a fluorimeter at a wavelength of 485 nm excitation and 530 nm emission.

*5.3 Evaluation of cell viability*

In order to verify that cell viability does not vary significantly in the presence of the tested product and under the experimental conditions, we performed a neutral red uptake assay (NRU) before and after irradiation of the cells. Neutral red (NR) is a weakly cationic dye that penetrates cell membrane by nonionic passive diffusion and concentrates in the lysosomes, where it binds by electrostatic hydrophobic bonds to anionic sites of the lysosomal matrix. The uptake of NR depends on the cell capacity to maintain pH gradients, through the production of ATP. Therefore NR accumulates only in alive cells, from which is extracted using an acidified ethanol solution: the absorbance of the solubilized dye is quantified using a spectrophotometer and is directly proportional to cell viability.

After having carefully removed the culture medium from each well, the cells were treated with neutral red medium (50 μg/ml) and incubated for 3 hours at standard culture conditions. Thereafter the plates were washed with PBS to eliminate dye wastes, and NR was extracted from live cells with an acidified ethanol solution. The plates were placed on a shaker for at least 10 minutes in order to dissolve the dye and the absorbance (optical density, OD) was determined spectrophotometrically at 540 nm wavelength.

*5.4 Results*

*5.4.1.Cell viability evaluation*

The absorbance measured at 540 nm is proportional to cell viability. Percentages were calculated based on the absorbance values at 540 nm and taking as 100% the absorbance of the negative control (untreated cells) (see table 6). In the absence of irradiation (UV-) cell viability of keratinocytes treated with the product is comparable to that of untreated keratinocytes for tested concentrations between 0.5 and 0.0156 mg/ml. Therefore these concentrations were taken into account in the dosage of ROS.

Table 6

| UV- | C+ | Sample (mg/ml) | | | | | | |
|---|---|---|---|---|---|---|---|---|
| | | 1.0 | 0.50 | 0.250 | 0.1250 | 0.0625 | 0.0313 | 0.0156 |
| | 74.7 | 66.5 | 87.7 | 104.8 | 110.3 | 107.7 | 106.6 | 105.0 |
| | | | | | | | | |
| UV+ | C+ | Sample (mg/ml) | | | | | | |
| | | 1.0 | 0.50 | 0.250 | 0.1250 | 0.0625 | 0.0313 | 0.0156 |
| | 107.7 | 76.0 | 84.3 | 90.2 | 96.3 | 95.9 | 99.9 | 103.9 |

*5.4.2 Antioxidant activity evaluation*

The fluorescence is directly proportional to the quantity of ROS produced by the cells. Percentages were calculated based on the measured fluorescence values and the percentage of reduction of ROS compared to the negative control (untreated cells). See table 7 below and figure 5.

The sample shows antioxidant activity (% reduction of ROS) at all tested concentrations and for all times of UV exposure.

Table 7

| ROS % Reduction | | | | | | | |
|---|---|---|---|---|---|---|---|
| UV irradiation times | C+ internal control | Sample (mg/ml) | | | | | |
| | | 0.5 | 0.25 | 0.125 | 0.0625 | 0.0313 | 0.0156 |
| (min) | | | | | | | |
| 4 | 78.6 | 48.8 | 41.5 | 36.3 | 31.2 | 31.0 | 29.8 |
| 8 | 80.3 | 41.3 | 32.5 | 29.7 | 24.7 | 24.2 | 28.8 |
| 12 | 72.4 | 36.8 | 26.9 | 24.4 | 20.4 | 19.2 | 25.0 |
| 16 | 64.6 | 35.1 | 24.7 | 21.3 | 16.3 | 14.9 | 20.8 |
| 20 | 52.5 | 29.5 | 19.8 | 18.0 | 14.4 | 12.9 | 17.2 |

*5.5 Conclusions*

The tested product reduces the ROS percentage in cell cultures of human keratinocytes exposed to oxidative stress. The sample shows antioxidant activity at tested concentration between 0.5 and 0.0156 mg/ml for all times of UV exposure.

Example 6 *In vivo* assays

The composition of the invention in the form a serum (see example 2) was applied twice a day, morning and night, over 28 days to 25 female volunteers with all skin types. The average values obtained were the following:

a) -57% total area of wrinkles measured by image analysis
b) +30% firmness measured by cutometry
c) +37% elasticity measured by elastimetry
d) +41% hydration measured by corneometry

For clarity purposes the following techniques have been explained in further detail:

Cutometry and elastimetry: The measurement of skin elasticity is based on the so-called suction method. A negative pressure is produced in the measuring head, and the skin is drawn inside the instrument. An optical measuring system consisting of a light source and light receptor measures the light intensity, which varies in accordance with the degree of skin penetration. The two parameters measured are firmness and elasticity. Firmness is measured in terms of the resistance that the skin displays against being drawn in by the negative pressure. Elasticity is measured in terms of the time taken for the skin to return to its original state.

Corneometry is a technology that is used to measure the hydration of the outer layer of the epidermis (stratum corneum). As the skin is a dielectric medium, variations in hydration show up through changes in capacity. In order to document the skin's moisture content, a measuring capacitor is pressed against the skin using constant pressure and the readings evaluated. The narrow diameter of the sensor even allows measurements to be taken on less accessible areas of skin. The corneometer is a fully automatic device. The reading indicates the epidermal hydration level - before and after treatment with cosmetic or pharmaceutical products, for instance. The recordings are always carried out within a constant time frame following the use of the respective product.

## Claims

**1.** Cosmetic or dermocosmetic composition comprising the following ingredients:

- extract of apple *(Malus domestica)* stem cells;
- extract of grape *(Solar vitis*) stem cells;
- at least one pentapeptide;
- a complex consisting of: epidermal growth factor, insulin-like growth factor, acid fibroblast growth factor, basic fibroblast growth factor vascular endothelial growth factor, vitamin B9 and glutamine.

**2.** Cosmetic or dermocosmetic composition, according to claim 1, wherein said at least one pentapeptide is pentapeptide-31 or pentapeptide-28 or a combination thereof.

**3.** Cosmetic or dermocosmetic composition, according to claim 1 or 2, wherein each of the ingredients is present in the following weight ratio with respect to the total composition:

- the extract of apple *(Malus domestica)* stem cells between 0.2 and 5 wt%;
- the extract of grape *(Solar vitis*) stem cells between 0.2 and 5 wt%;
- the at least one pentapeptide between 0.2 and 5 wt% for each pentapeptide;
- the complex between 0.2 and 5 wt%.

**4.** Cosmetic or dermocosmetic composition, according to any of claims 1 to 3, further comprising at least one of the following ingredients:

- apricot extract;
- panthenol;
- lotus flower extract;
- hyaluronic acid;
- viola tricolor extract;
- polyoses from oats extract.

**5.** Product comprising the composition according to any of the preceding claims.

**6.** Product, according to claim 5, wherein the composition is present in a concentration between 0.01% and 5% by weight with respect to the total weight of the product.

**7.** Product, according to claim 5 or 6, which is in the form of an emulsion with oil as the external phase (W/O).

**8.** Product, according to claim 5 or 6, which is in the form of an oil-in-water dispersion without emulsifier.

**9.** Product, according to claim 5 or 6, which is in the form of an emulsion with water as the external phase (O/W).

**10.** Product, according to claim 5 or 6, which is in the form of a solution.

**11.** Product, according to claim 5 or 6, which is in the form of a gel.

**12.** Process for preparing a composition, according to any of claims 1 to 4, comprising the step of mixing all the ingredients with simple and/or fast agitation.

**13.** Process for preparing the product, according to claim 7, comprising the steps of:

a) heating the oil phase at 75-80°C,
b) heating the aqueous phase at 75-80°C,
c) pouring the aqueous phase over the oil phase with agitation and shear;
d) cooling with slow agitation.

**14.** Process for preparing the product, according to claim 8 or 9, comprising the steps of:

a) heating the aqueous phase at 75-80°C,
b) heating the oil phase at 75-80°C,
c) pouring the oil phase over the aqueous phase with agitation and shear;
d) cooling with slow agitation.

**15.** Use of the composition, according to any of claims 1 to 4, or the product, according to any of claims 5 to 11, for treating the aging of the skin.

Figure 1

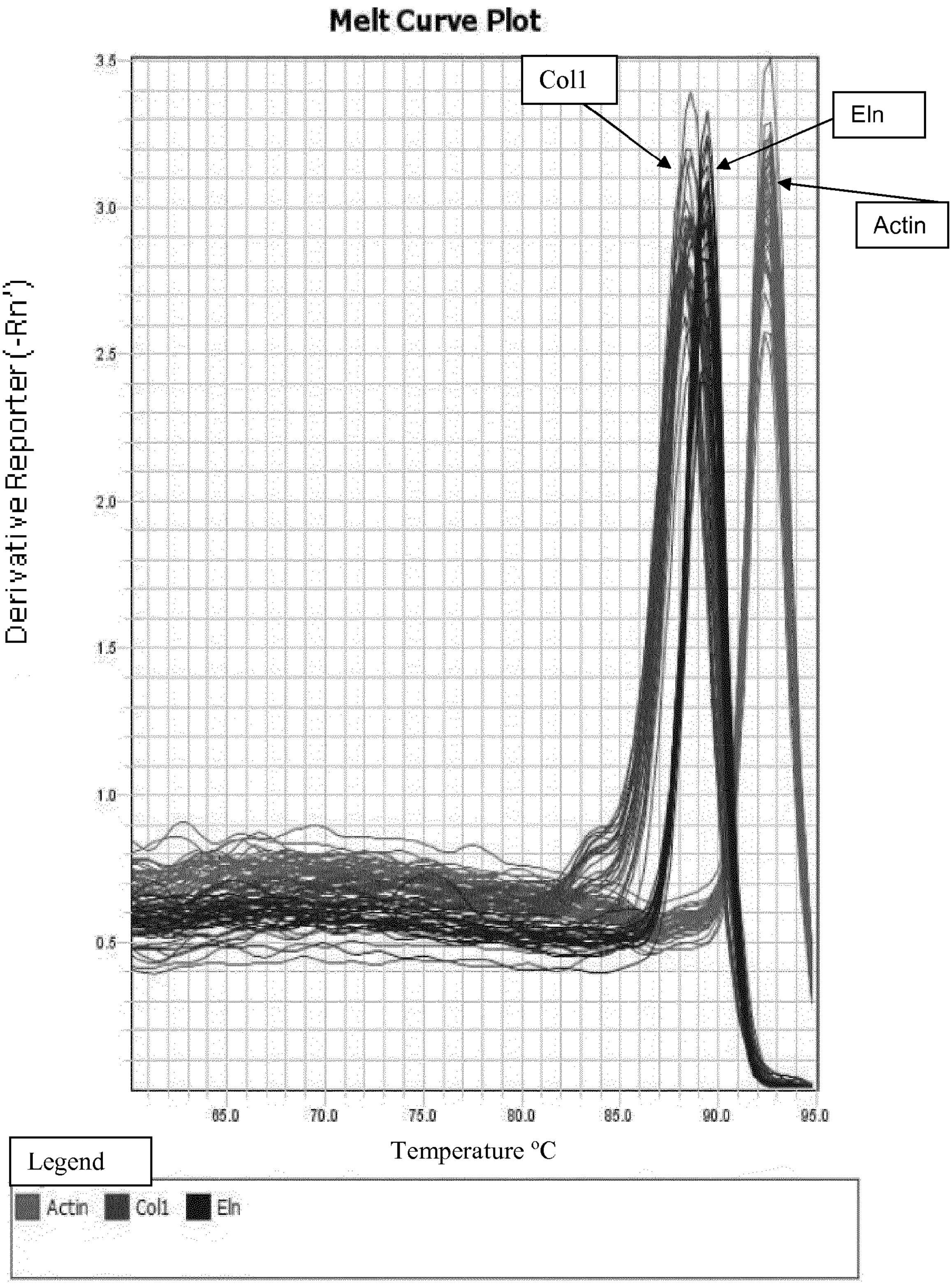
Melt Curve Plot
Col1
Eln
Actin
3.5
3.0
2.5
2.0
1.5
1.0
0.5
Derivative Reporter (-Rn')
65.0
70.0
75.0
80.0
85.0
90.0
95.0
Temperature °C
Legend
Actin
Col1
Eln

Figure 2A

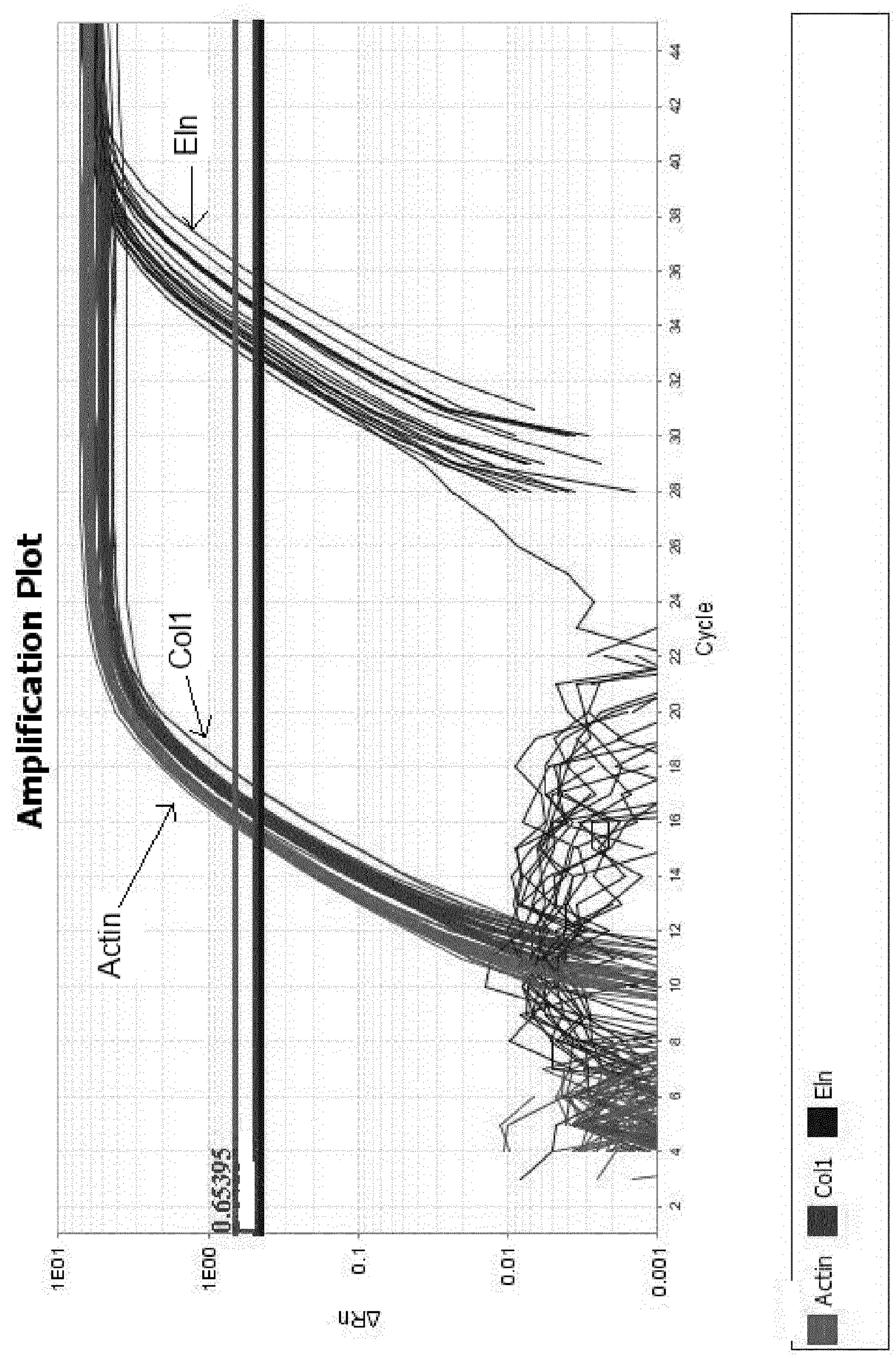
Amplification Plot
Actin
Col1
Eln
0.65395
1E01
1E00
0.1
0.01
0.001
ΔRn
Cycle
2
4
6
8
10
12
14
16
18
20
22
24
26
28
30
32
34
36
38
40
42
44
Actin
Col1
Eln

Figure 2B

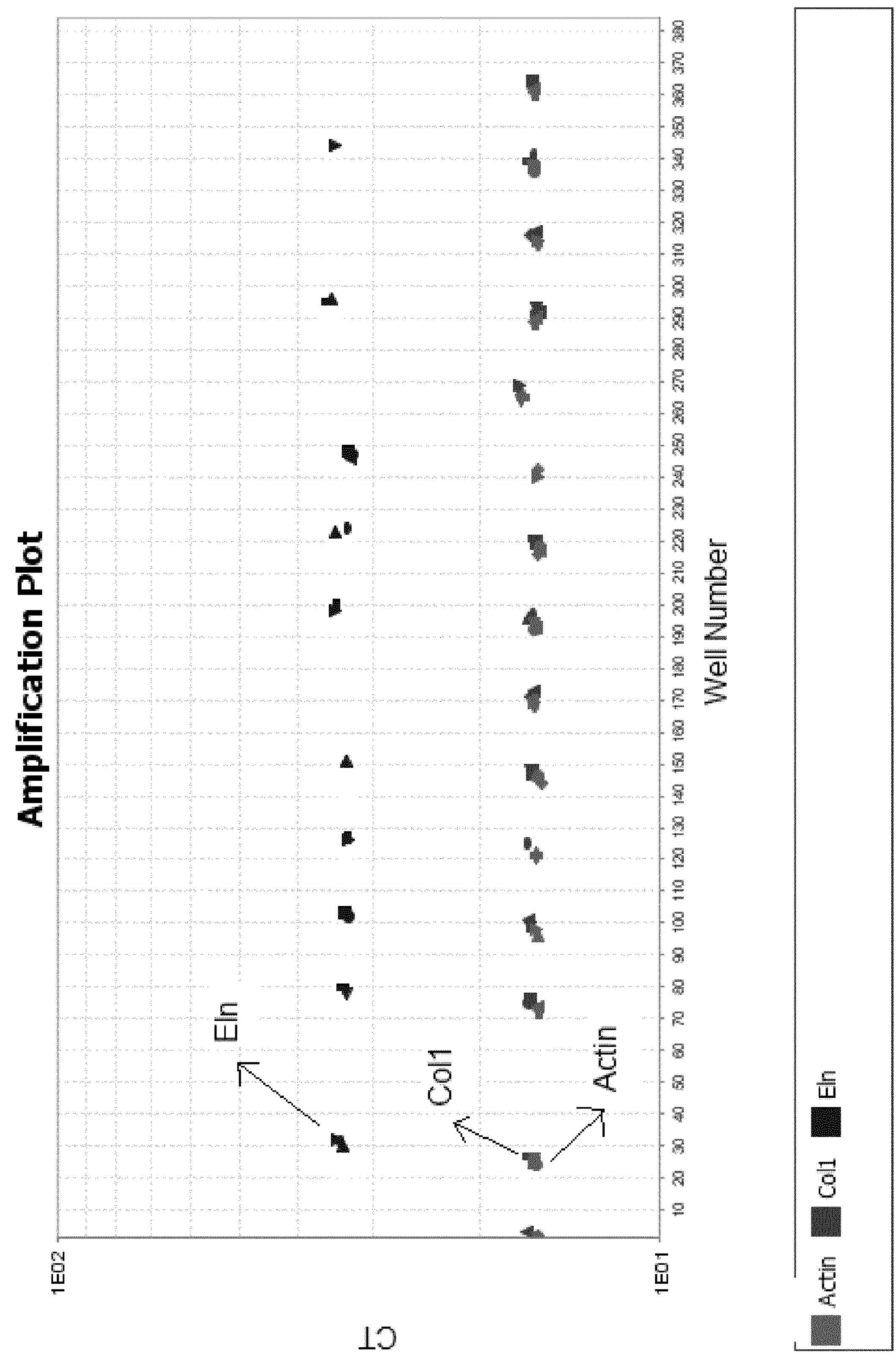
Amplification Plot
CT
1E02
1E01
Well Number
10
20
30
40
50
60
70
80
90
100
110
120
130
140
150
160
170
180
190
200
210
220
230
240
250
260
270
280
290
300
310
320
330
340
350
360
370
380
Eln
Col1
Actin
Actin
Col1
Eln

Figure 2C

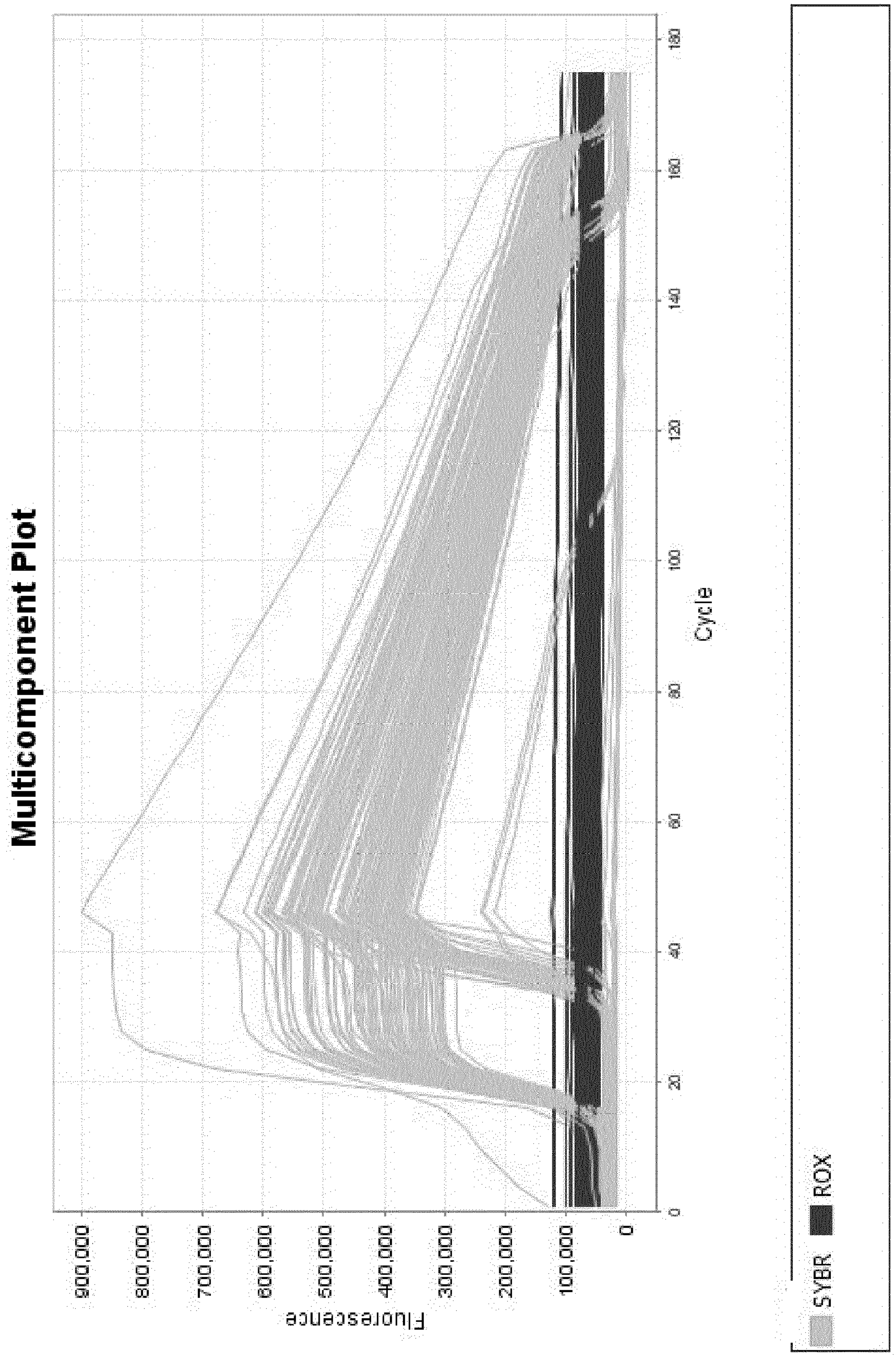
Multicomponent Plot
Fluorescence
900,000
800,000
700,000
600,000
500,000
400,000
300,000
200,000
100,000
0
Cycle
0
20
40
60
80
100
120
140
160
180
SYBR
ROX

Figure 3

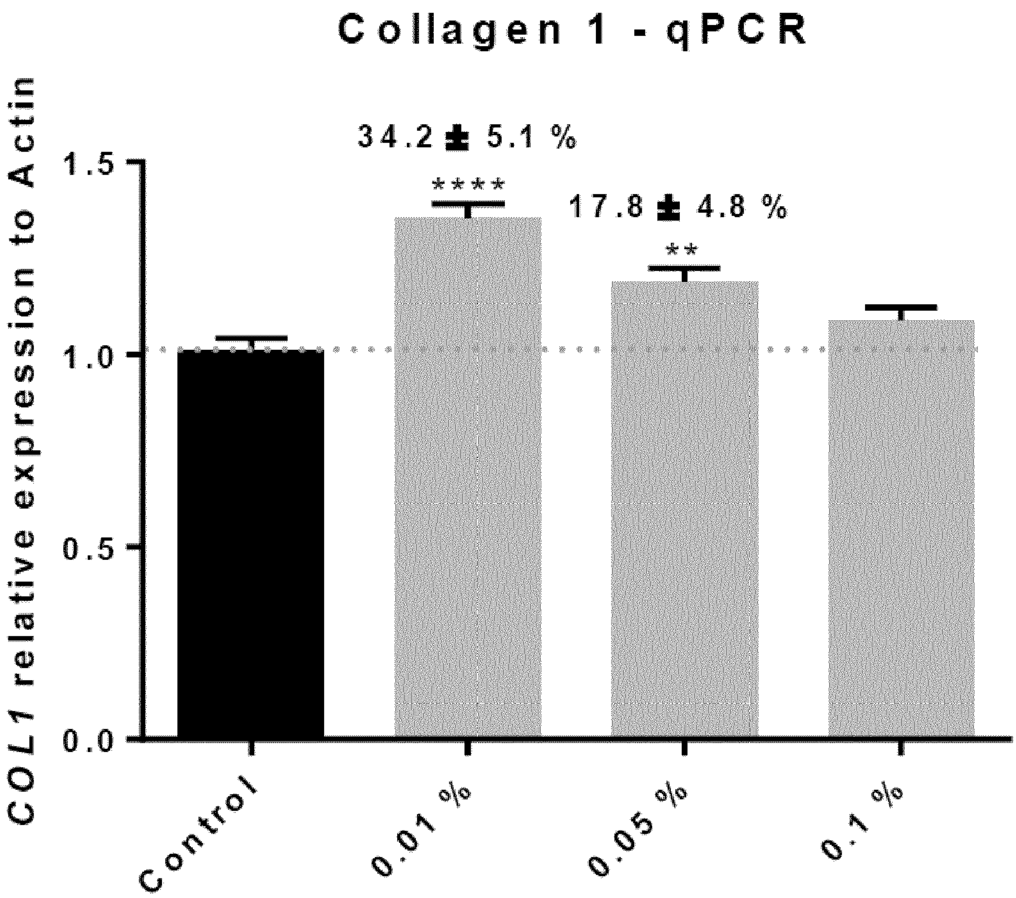
Collagen 1 - qPCR
COL1 relative expression to Actin
1.5
1.0
0.5
0.0
34.2 ± 5.1 %
****
17.8 ± 4.8 %
**
Control
0.01 %
0.05 %
0.1 %

Figure 4

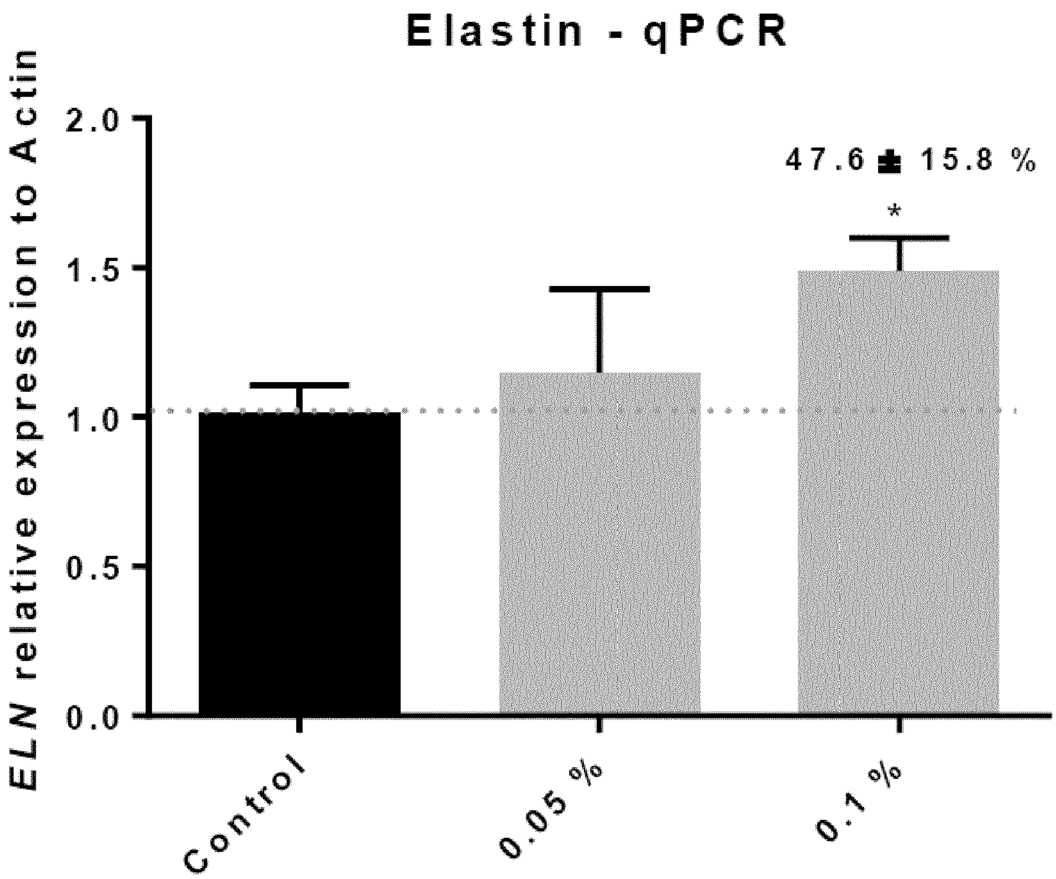
Elastin - qPCR
ELN relative expression to Actin
2.0
1.5
1.0
0.5
0.0
47.6 ± 15.8 %
*
Control
0.05 %
0.1 %

Figure 5

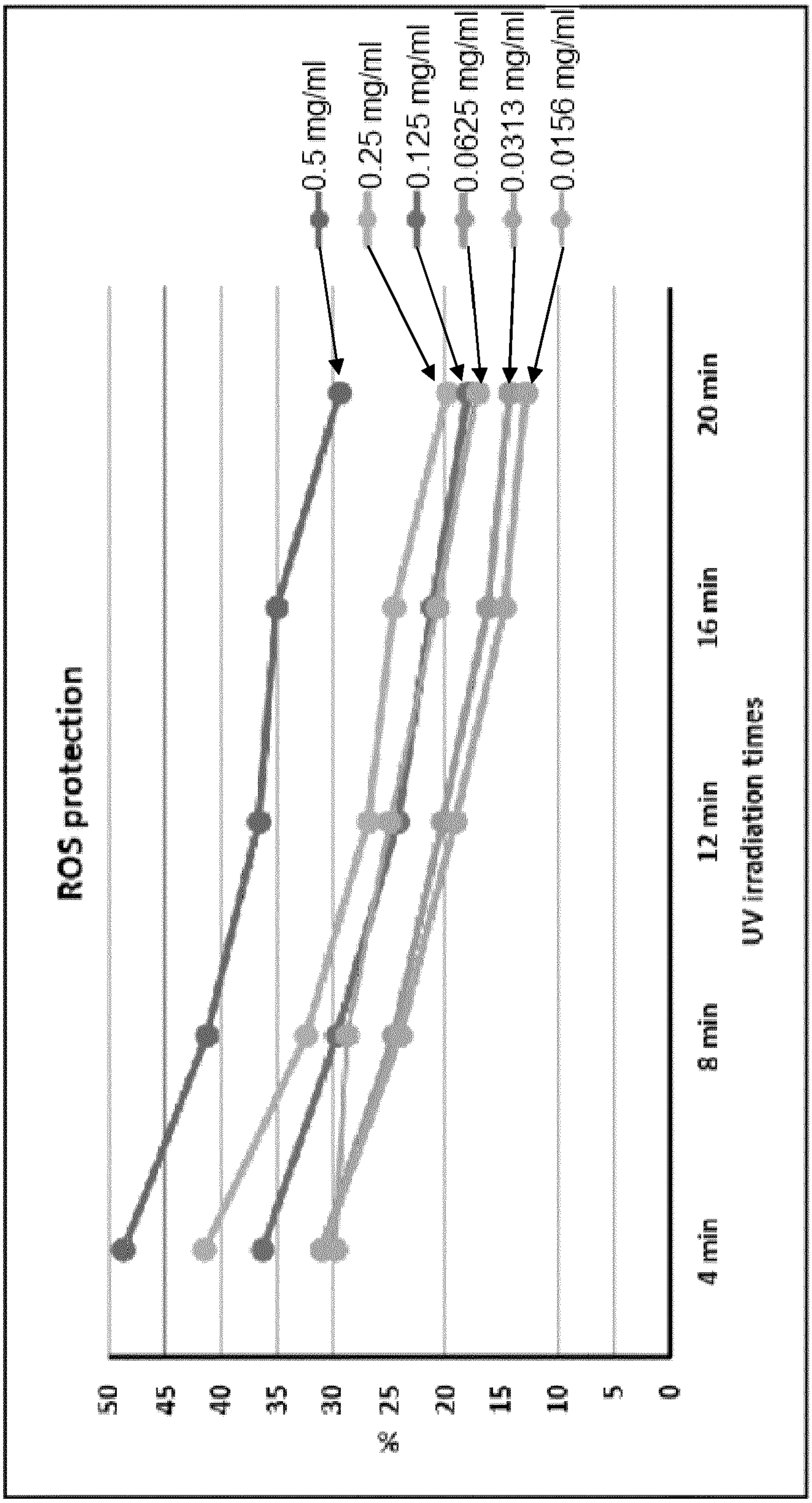

ROS protection
%
50
45
40
35
30
25
20
15
10
5
0
4 min
8 min
12 min
16 min
20 min
UV irradiation times
0.5 mg/ml
0.25 mg/ml
0.125 mg/ml
0.0625 mg/ml
0.0313 mg/ml
0.0156 mg/ml

Europäisches Patentamt
European Patent Office
Office européen des brevets

# EUROPEAN SEARCH REPORT

Application Number
EP 17 38 2594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/224150 A1 (CHUNG YONGJI [US]) 27 September 2007 (2007-09-27) * paragraphs [0004] - [0028], [0052], [0055]; examples * ----- | 1-15 | INV. A61K8/64 A61Q19/08 A61K8/97 |
| Y | KR 2017 0049299 A (INDUSTRY-ACADEMIC COOP FOUND GYEONGSANG NAT UNIV [KR]; HUMAN BIOTECH C) 10 May 2017 (2017-05-10) * paragraphs [0001] - [0015]; examples * ----- | 1-15 | |
| Y | WO 2015/107237 A1 (SANI RED S L [ES]) 23 July 2015 (2015-07-23) * paragraphs [0004] - [0007], [0014], [0024] - [0030] * ----- | 1-15 | |
| Y | MIBELLEBIOCHEMISTRY: "PhytoCellTec Malus Domestica - Plant stem cells to protect skin stem cells", INTERNET CITATION, 2008, pages 1-12, XP002758104, Retrieved from the Internet: URL:https://mibellebiochemistry.com/products/phytocelltec-malus-domestica/ [retrieved on 2016-05-27] * the whole document * ----- | 1-15 | TECHNICAL FIELDS SEARCHED (IPC) A61K A61Q |
| Y | Unknown: "PhytoCellTec(TM) Solar Vitis Protects epidermal stem cells against UV damage", , 23 September 2009 (2009-09-23), pages 1-8, XP055434608, Retrieved from the Internet: URL:https://biogreenscience.com/file/clinical/Solar_Vitis.pdf [retrieved on 2017-12-13] * the whole document * ----- -/-- | 1-15 | |

The present search report has been drawn up for all claims

1

| Place of search | Date of completion of the search | Examiner |
|---|---|---|
| Munich | 13 December 2017 | Steffen, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 17 38 2594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Unknown: "Vincience Biofunctionals - 2016 synopsis for skin care and hair care applications", Ashland , 24 October 2016 (2016-10-24), pages 1-20, XP002776666, Retrieved from the Internet: URL:http://www.ashland.com/file_source/Ashland/Industries/Personal%20and%20Home%20Care/Articles/PC-12152.11_Vincience.pdf [retrieved on 2017-12-13] * pages 5,14 * | 1-15 | |
| Y | Anne Curtis: "New Developments in the Science Behind Anti-Aging Skin Care Products", Skin Therapy Letter (vol. 10(3), November 2014), 21 October 2014 (2014-10-21), pages 1-12, XP055434670, Retrieved from the Internet: URL:http://www.skintherapyletter.com/fp/download/stl_fp_10_3_en.pdf [retrieved on 2017-12-13] * page 8 * | 1-15 | TECHNICAL FIELDS SEARCHED (IPC) |
| Y | Unknown: "BIO-Placenta", , 9 November 2015 (2015-11-09), pages 1-2, XP055434631, Retrieved from the Internet: URL:http://www.independentchemical.com/adminimg/productpdf/1544256163bio-placenta.pdf [retrieved on 2017-12-13] * the whole document * | 1-15 | |

-/--

1

The present search report has been drawn up for all claims

| Place of search | Date of completion of the search | Examiner |
|---|---|---|
| Munich | 13 December 2017 | Steffen, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 38 2594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; August 2017 (2017-08), Unknown: "Legacy Youth Treatment", XP002776667, Database accession no. 5041051 * the whole document * ----- | 1-15 | |

TECHNICAL FIELDS SEARCHED (IPC)

1

The present search report has been drawn up for all claims

| Place of search | Date of completion of the search | Examiner |
|---|---|---|
| Munich | 13 December 2017 | Steffen, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 38 2594

This annex lists the patent family members relating to the patent documents cited in the above-mentioned European search report. The members are as contained in the European Patent Office EDP file on
The European Patent Office is in no way liable for these particulars which are merely given for the purpose of information.

13-12-2017

| Patent document cited in search report | Publication date | Patent family member(s) | Publication date |
|---|---|---|---|
| US 2007224150 A1 | 27-09-2007 | NONE | |
| KR 20170049299 A | 10-05-2017 | NONE | |
| WO 2015107237 A1 | 23-07-2015 | ES 2541177 A2 | 16-07-2015 |
| | | WO 2015107237 A1 | 23-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **UITTO, J.** The role of elastin and collagen in cutaneous aging: intrinsic aging versus photoexposure. *J Drugs Dermatol.,* 2008, vol. 7 (2), S12-S16
- **SARDY, M.** Role of matrix metalloproteinases in skin ageing. *Connect Tissue Res.,* 2009, vol. 50 (2), 132-138
- **PFAFFL, M.** A new mathematical model for relative quantification in real-time RT-PCR. *Nucleic Acids Research,* 2001, vol. 29 (9